# EUROPEAN PATENT APPLICATION

(11) **EP 4 431 615 A1**
(43) Date of publication of application: **18.09.2024**
(21) Application number: 22893110.1
(22) Date of filing: 03.11.2022
(51) Int. Cl.: C12P 23/00, C12N 9/02, C07K 14/245, C12N 15/70, C12N 9/12, C12N 9/10

(54) **COMPOSITION FOR PREPARING RETINOID, AND METHOD FOR PREPARING RETINOID USING SAME**

(30) Priority: 09.11.2021 KR 20210153276
(71) Applicant: Industry-Academic Cooperation Foundation Gyeongsang National University, Jinju-si, Gyeongsangnam-do 52828 (KR)
(72) Inventor: KIM, Seon Won, Sejong 30062 (KR); PARK, Ji Bin, Busan 46277 (KR); JEONG, Seong Hee, Gimhae-si, Gyeongsangnam-do 50803 (KR); KWON, Moon Hyuk, Jinju-si, Gyeongsangnam-do 52831 (KR)
(74) Representative: Dragotti & Associati S.R.L.
(86) International application number: PCT/KR2022/017114
(87) International publication number: WO 2023/085688

(57) **Abstract**

The present invention relates to a composition for preparing retinoid, and more specifically, the composition comprises: a NADH or NADPH, a Beta-Carotene Oxygenase and a flavodoxin or a ferredoxin or a reductase of flavodoxin or ferredoxin; or a microorganism expressing them; or a culture or pulverized product of the microorganism, and thus can expand the supply metabolism of reducing electrons to enhance retinoid productivity through an increase in the activity of the cytochrome P450 system and provide a foundation for the low-cost production of raw materials, and thereby enhance the economic feasibility and efficiency of retinoid production.

## Description

### [Technical field]

The present invention relates to a composition for producing retinoid and a retinoid producing method using the same.

### [Background]

Retinoids are a class of lipophilic isoprenoid molecules that are chemically related to vitamin A. Retinoids consist of retinol, retinal, retinoic acid, or retinyl acetate. They are necessary to maintain three basic physiological functions: vision protection, growth and development, and immunity, and are known to reduce the risk of certain cancers. In recent years, retinoids have gained increasing attention as a pharmaceutical and functional cosmetic ingredient for the treatment of wrinkles and skin diseases by increasing skin elasticity, resulting in rapidly growing industrial demand and an estimated $4.3 billion global market.

The monooxygenation reaction, the main enzymatic reaction of beta-carotene monooxygenase (Beta-Carotene 15,15'-MonoOxygenase: BCMO), a cytochrome P450 enzyme that produces retinal from beta-carotene, requires an oxygen molecule (O₂) and a reducing substance of NADPH (or NADH), where one atom of the oxygen molecule binds to the substrate being oxidized and the other atom is reduced to water. Cytochrome P450 enzymes in microorganisms such as bacteria and mitochondria use a two-component shuttle system consisting of flavodoxin, ferredoxin, flavodoxin reductase, and ferredoxin reductase as an electron transfer partner.

In previous prior art, enhanced retinoid production using microorganisms has been studied, but retinoids are chemically unstable, containing reactive conjugated double bonds, and are easily oxidized and isomerized by heat, oxygen, and light, resulting in low production efficiency. In order to actually utilize retinoids for industrial production, further research is required to improve production efficiency so that they can be economically produced in large quantities. Therefore, it is necessary to develop biotechnological methods to increase the efficiency of retinoid conversion by increasing the activity of the cytochrome P450 system to improve productivity.

### [Detailed description of the invention]

### [Technical problem]

The present invention aims to provide compositions for the manufacture of retinoids with increased production efficiency.

The present invention aims to provide a method for the preparation of retinoids with increased production efficiency.

### [Technical solution]

1. A composition for production of retinoid comprising a NADH or NADPH, a Beta-Carotene Oxygenase and a flavodoxin or a ferredoxin or reductase of flavodoxin or ferredoxin; or a microorganism expressing them; or a culture or pulverized product of the microorganism.
2. The composition for production of retinoid according to claim 1, wherein the Beta-Carotene Oxygenase is a Beta-Carotene MonoOxygenase(BCMO, Beta-Carotene 15,15'-MonoOxygenase) comprising a sequence of SEQ ID NO: 1.
3. The composition for production of retinoid according to claim 1, wherein the flavodoxin is a flavodoxin 1(fldA) comprising a sequence of SEQ ID NO: 2 or a flavodoxin 2(fldB) comprising a sequence of SEQ ID NO: 3.
4. The composition for production of retinoid according to claim 1, wherein the ferredoxin is a reduced ferredoxin(fdx) comprising a sequence of SEQ ID NO: 4.
5. The composition for production of retinoid according to claim 1, wherein the flavodoxin or ferredoxin reductase is a flavodoxin/ferredoxin-NADP(+) reductase(fpr) comprising a sequence of SEQ ID NO: 5 or a pyruvate-flavodoxin oxidoreductase(YdbK) comprising a sequence of SEQ ID NO: 6.
6. The composition for production of retinoid according to claim 1, wherein the microorganism is of the genus *Escherichia.*
7. The composition for production of retinoid according to claim 1, wherein the microorganism further expresses a gene encoding an enzyme of a mevalonate(MVA) synthesis pathway or a beta-carotene synthesis pathway.
8. The composition for production of retinoid according to claim 7, wherein the enzyme of the mevalonate synthesis pathway is an acetyl-CoA acetyltransferase/hydroxymethylglutaryl (HMG)-COA reductase(mvaE) comprising a sequence of SEQ ID NO: 18, a HMG-CoA synthase(mvaS) comprising a sequence of SEQ ID NO: 19, a mevalonate kinase(mvaK1) comprising a sequence of SEQ ID NO: 20, a phosphomevalonate kinase(mvaK2) comprising a sequence of SEQ ID NO: 21, a mevalonate diphosphate decarboxylase(mvaD) comprising a sequence of SEQ ID NO: 22, and an isopentenyldiphosphate isomerase(idi) comprising a sequence of SEQ ID NO: 23.
9. The composition for production of retinoid according to claim 7, wherein the enzyme of the beta-carotene synthesis pathway is a geranylgeranyl pyrophosphate synthase(crtE) comprising a sequence of SEQ ID NO: 48, a phytoene synthase(crtB) comprising a sequence of SEQ ID NO: 49, a phytoene dehydrogenase(crtl) comprising a sequence of SEQ ID NO: 50, a lycopene beta-cyclase(crtY) comprising a sequence of SEQ ID NO: 51, an IPP isopomerase(isopentenyl pyrophosphate:dimethylallyl pyrophosphate isomerase, ipiHP1) comprising a sequence of SEQ ID NO: 52.
10. The composition for production of retinoid according to claim 1, wherein the microorganism where at least one gene encoding an enzyme selected from a group consisting of ackA(acetate kinase), pta(phosphate acetyltransferase/phosphate propionyltransferase), adhE(aldehyde-alcohol dehydrogenase), IdhA(lactate dehydrogenase A), atoDA(acyl CoA:acetate/3-ketoacid CoA transferase), dld(D-lactate dehydrogenase), poxB(pyruvate dehydrogenase) and pps(phosphoenolpyruvate synthase) is attenuated or deleted.
11. A method for production of retinoid comprising reacting a beta-carotene with the composition according to claim 1.
12. The method for production of retinoid according to claim 11, wherein the composition comprises the microorganism, and the reaction is carried out within the microorganism.
13. The method for production of retinoid according to claim 11, wherein the composition comprises the microorganism, and the method further comprises culturing the microorganism in a medium containing a carbon source.

### [Effect of the invention]

The present invention can improve the economics and efficiency of retinoid production by expanding the reducing electron supply metabolism and increasing the activity of the cytochrome P450 system to improve retinoid productivity and establish a foundation for low-cost raw material production.

### [Brief description of the drawings]

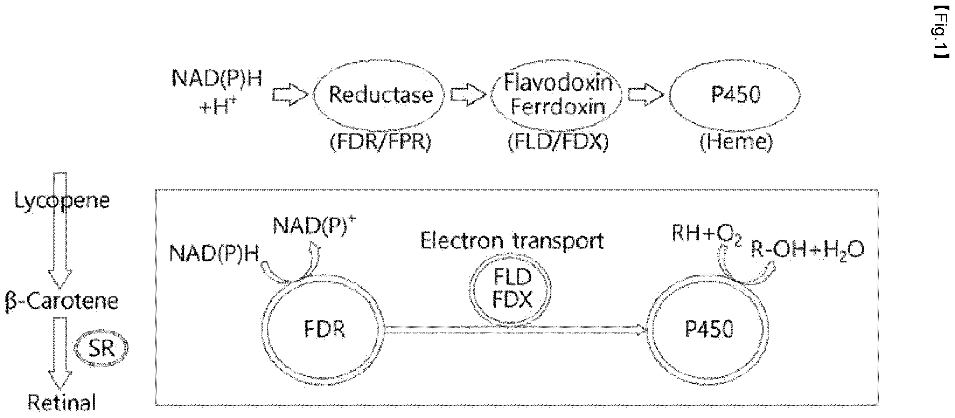

FIG. 1 is a schematic diagram of the retinal synthesis process and the electron transport system involved in it.
FIG. 2 is a High Performance Liquid Chromatography (HPLC) analysis profile of retinoids including retinal, retinol, and retinyl acetate.
FIG. 3 is a diagram schematically showing the MEP pathway and the exogenous MVA pathway of retinal biosynthesis.
FIG. 4 schematically shows the SBL1 strain production process.
FIG. 5 schematically shows the SBL7 strain production process.
FIG. 6 shows cell growth of retinoid producing strain MG1655 into which pB2-fB, pB2-fr, pB2-fAfr, pB2-fBfr or pB2-fAyK was introduced.
FIG. 7 shows the retinoid production amount of retinoid producing strain MG1655 into which pB2-fB, pB2-fr, pB2-fAfr, pB2-fBfr or pB2-fAyK was introduced.
FIG. 8 shows cell growth of the retinoid producing strain AceCo into which pB2-fA, pB2-fB, pB2-fr, pB2-yK, pB2-fAfr, pB2-fBfr, pB2-fAyK, or pB2-fByK was introduced.
FIG. 9 shows the retinoid production of the retinoid producing strain AceCo into which pB2-fA, pB2-fB, pB2-fr, pB2-yK, pB2-fAfr, pB2-fBfr, pB2-fAyK, or pB2-fByK was introduced.

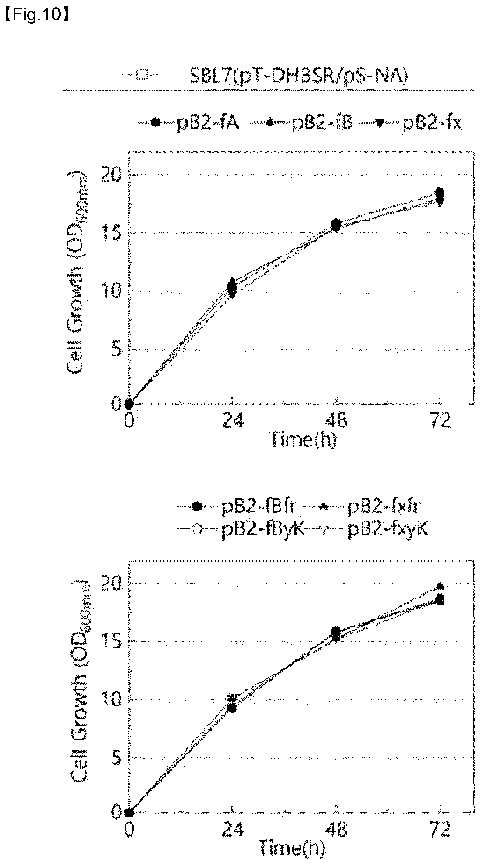

FIG. 10 shows cell growth of retinoid producing strain SBL7 into which pB2-fA, pB2-fB, pB2-fx, pB2-fBfr, pB2-fxfr, pB2-fByK, or pB2-fxyK was introduced.

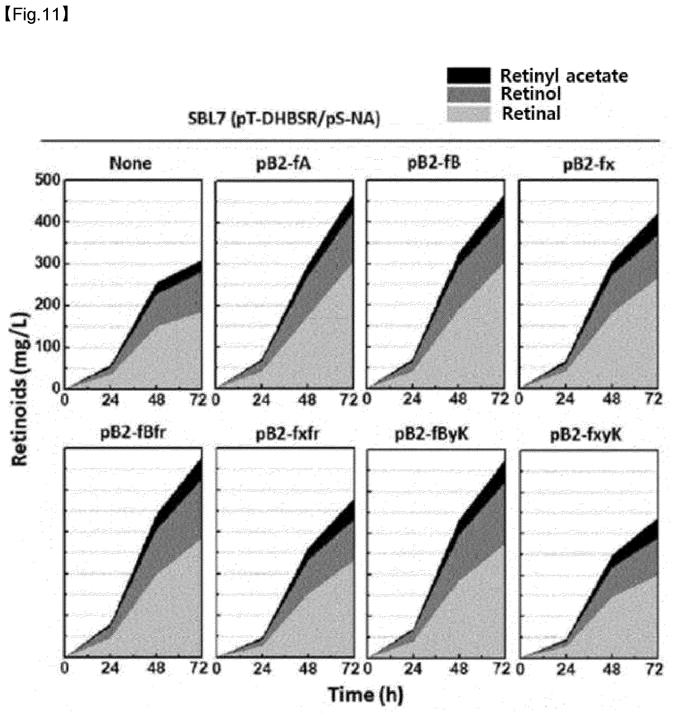

FIG. 11 shows the retinoid production of the retinoid producing strain SBL7 into which pB2-fA, pB2-fB, pB2-fx, pB2-fBfr, pB2-fxfr, pB2-fByK, or pB2-fxyK was introduced.

### [Form for practicing the invention]

Hereinafter, the present invention will be described in detail.

The present invention provides a composition for producing retinoids.

The composition for producing retinoids includes a NADH or NADPH, a beta-carotene oxidase (Beta-Carotene Oxygenase) and a flavodoxin or a ferredoxin or a reductase of flavodoxin or ferredoxin; or a microorganism expressing them; or a culture or pulverized product of the microorganism.

Retinoids may be compounds with the skeleton of vitamin A (retinol) or compounds that bind to retinoic acid receptors with the same or similar effects as retinoic acid (all trans and 9-cis), the activator of retinol. The retinoid may be, for example, retinal, retinol, retinyl palmitate, retinoic acid, or retinyl acetate, and may specifically be retinal, but is not limited thereto.

Beta-carotene oxidase is an enzyme that catalyzes the reaction that produces two molecules of retinal from beta-carotene, the precursor of vitamin A (retinol). The beta-carotene oxidation enzyme may be, for example, beta-carotene monooxygenase (BCMO, Beta-Carotene 15,15'-MonoOxygenase). Beta-carotene monooxygenase may include a sequence of SEQ ID NO: 1, but is not limited thereto.

Flavodoxin is an electron transfer enzyme, which provides electrons or hydrogen ions used as a driving force through cofactors such as NAD and NADP to enzymes involved in oxidation or reduction reactions in vivo. means. Flavodoxin is used as an electron transfer partner in the reaction that generates two molecules of retinal from beta-carotene. The flavodoxin may be a flavodoxin 1 (fldA) or a flavodoxin 2 (fldB). Flavodoxin 1 may include a sequence of SEQ ID NO: 2, but is not limited thereto. Flavodoxin 2 may include a sequence of SEQ ID NO: 3, but is not limited thereto.

Ferredoxin is an electron transfer enzyme that is used as an electron transfer partner in the reaction that produces two molecules of retinal from beta-carotene. Ferredoxin may be a reduced ferredoxin (fdx). The reduced ferredoxin may include a sequence of SEQ ID NO: 4, but is not limited thereto.

Flavodoxin or ferredoxin reductase is an electron transfer enzyme that is used as an electron transfer partner in the reaction that produces two molecules of retinal from beta-carotene. The reductase enzyme for flavodoxin or ferredoxin is flavodoxin/ferredoxin-NADP(+) reductase (fpr) or pyruvate-flavodoxin oxidoreductase. oxidoreductase, YdbK). The flavodoxin/ferredoxin-NADP(+) reductase enzyme may include a sequence of SEQ ID NO: 5, but is not limited thereto. The pyruvic acid-flavodoxin oxidoreductase enzyme may include a sequence of SEQ ID NO: 6, but is not limited thereto.

Flavodoxin or ferredoxin, or a reductase of flavodoxin or ferredoxin, may optionally be included. For example, only flavodoxin, only ferredoxin, only flavodoxin reductase, or only ferredoxin reductase may be included. A combination of at least two of these may be included. For example, flavodoxin and its reductase may be included, flavodoxin and ferredoxin reductase may be included, and ferredoxin and flavodoxin reductase may be included.

The microorganism expressing these may express them intrinsically or exogenously. Microorganism expressing them exogenously may be a microorganism into which genes encoding NADH or NADPH, beta-carotene oxidase (Beta-Carotene Oxygenase), flavodoxin or ferredoxin, or reductase of flavodoxin or ferredoxin are introduced. Gene introduction may be carried out using viral vectors such as plasmids, retroviruses, adenoviruses, and non-viral vectors known in the art, without limitation.

Microorganism may be a prokaryotic cell or eukaryotic cell that can be cultured in a liquid medium. The microorganism may be, for example, a bacterium, fungus, or a combination thereof. Bacteria may be Gram-positive bacteria, Gram-negative bacteria, or a combination thereof, and to increase psicose production capacity, bacteria may be Gram-positive bacteria. Gram-negative bacteria may include the genus Escherichia. Gram-positive bacteria may be the genus Bascillus, the genus Corynebacterium, the genus Actinomyces, the genus Lactobacillus or a combination thereof. Fungi may be yeast, the genus Kluyveromyces or a combination thereof. The microorganism may be a natural or foreign gene introduced. The foreign gene may be a gene involved in retinoid production, such as one or more genes of the MEP or MVA pathway.

The microorganism may be a microorganism of the genus Escherichia, specifically Escherichia coli (Escherichia coli), and more specifically an Escherichia coli MG1655 strain, but is not limited thereto.

The culture of the microorganism may include a medium containing microorganisms after culture, a medium in which microorganisms are separated after culture, or substances secreted by microorganisms during culture. The medium may be a solid medium or a liquid medium. The crushed product of the microorganism is the crushed product of the microorganism through sonication, etc., and may include the protein in the microorganism.

Microorganisms may further express genes encoding enzymes of the mevalonate (MVA) synthesis pathway or beta-carotene synthesis pathway.

In the case of microorganism further expressing genes encoding the mevalonate synthesis pathway or enzymes of the beta-carotene synthesis pathway, the production efficiency of mevalonate or lycopene or beta-carotene, which are precursors of beta-carotene, is improved so that retinoids can be obtained with excellent yield when using the microorganism as a composition for the manufacture of retinoids.

The microorganism may endogenously or exogenously express genes encoding enzymes of the mevalonate synthesis pathway or the beta-carotene synthesis pathway. Microorganism that exogenously expresses these may have genes encoding enzymes of the mevalonate synthesis pathway or beta-carotene synthesis pathway introduced. Gene introduction may be carried out using viral vectors such as plasmids, retroviruses, adenoviruses, and non-viral vectors known in the art, without limitation.

The enzymes in the mevalonate synthesis pathway may be acetyl-CoA acetyltransferase/hydroxymethylglutaryl (HMG)-CoA reductase (mvaE) and HMG-CoA synthase (mvaS), mevalonate kinase (mvaK1), phosphomevalonate kinase (mvaK2), diphosphomevalonate decarboxylase (mvaD), and isopentenyldiphosphate isomerase (idi).

The mvaE may include a sequence of SEQ ID NO: 18, but is not limited thereto. The mvaS may include a sequence of SEQ ID NO: 19, but is not limited thereto. The mvaK1 may include a sequence of SEQ ID NO: 20, but is not limited thereto. The mvaK2 may include a sequence of SEQ ID NO: 21, but is not limited thereto. The mvaD may include a sequence of SEQ ID NO: 22, but is not limited thereto. The idi may include a sequence of SEQ ID NO: 23, but is not limited thereto.

The mvaE may be derived from *Enterococcus faecalis.* The mvaS may be derived from *Enterococcus faecalis.* The mvaK1 may be derived from *Streptococcus pneumoniae.* The mvaK2 may be derived from *Streptococcus pneumoniae.* The mvaD may be derived from *Streptococcus pneumoniae.* The idi may be derived from *Escherichia coli.*

The enzymes in the beta-carotene synthesis pathway may be geranylgeranyl pyrophosphate synthase (crtE), phytoene synthase (crtB), phytoene dehydrogenase (crtl), lycopene beta-cyclase (crtY) and IPP isopomerase (isopentenyl pyrophosphate:dimethylallyl pyrophosphate isomerase, ipiHP1).

The crtE may include a sequence of SEQ ID NO: 48, but is not limited thereto. The crtB may include a sequence of SEQ ID NO: 49, but is not limited thereto. The crtl may include a sequence of SEQ ID NO: 50, but is not limited thereto. The crtY may include a sequence of SEQ ID NO: 51, but is not limited thereto. The ipiHP1 may include a sequence of SEQ ID NO: 52, but is not limited thereto.

The crtE may be derived from *Pantoea agglomerans.* The crtB may be derived from *Pantoea aglumerans.* The crtl may be derived from *Pantoea aglumerans.* The crtY may be derived from *Pantoea ananatis.* The ipiHP1 may be derived from *Haematococcus pluvialis.*

Microorganism may have genes encoding enzymes that produce fermentation by-products attenuated or deleted. During the retinoid production reaction, acetate, alcohol, lactate, acetoacetate, phosphoenolpyburic acid, etc. may be produced as by-products of fermentation, which consumes acetyl-CoA, a precursor in the mevalonate synthesis pathway, and retinoid production efficiency decreases. If the gene encoding the enzyme that produces fermentation by-products is attenuated or deleted, unnecessary consumption of the precursor acetyl-CoA can be prevented and retinoid productivity can be maximized.

The enzymes that produce fermentation by-products may be at least one selected from a group consisting of ackA (acetate kinase), pta (phosphate acetyltransferase/phosphate propionyltransferase), adhE (aldehyde-alcohol dehydrogenase), IdhA (lactate dehydrogenase A), atoDA (acyl CoA:acetate/3-ketoacid CoA transferase), did (D-lactate dehydrogenase), poxB (pyruvate dehydrogenase), and pps (phosphoenolpyruvate synthase).

The ackA may be an acetate kinase involved in acetate production. The pta may be a phosphate acetyltransferase/phosphate propionyltransferase, which is involved in acetate production. The adhE may be an aldehyde-alcohol dehydrogenase involved in alcohol production. The IdhA may be a lactate dehydrogenase A, which is involved in lactate production. The atoDA may be acyl CoA:acetate/3-ketoacid CoA transferase involved in acetoacetate production. The did may be a D-lactate dehydrogenase, which is involved in lactate production. The poxB may be a pyruvate dehydrogenase, which is involved in acetate production. The pps may be a phosphoenolpyruvate synthase, which is involved in producing phosphoenolpyruvate.

The present invention provides a method for producing retinoids.

The method for producing a retinoid includes reacting beta-carotene with the composition for producing a retinoid.

Beta-carotene, retinoids, and compositions for producing retinoids may be within the above-mentioned range, but are not limited thereto.

When beta-carotene is reacted with the composition for producing retinoid, a reaction that generates two molecules of retinal from beta-carotene is activated, so that retinoid can be produced with excellent yield.

When the composition for producing a retinoid includes the microorganism, the reaction between beta-carotene and the composition for producing a retinoid may be carried out within the microorganism.

Microorganisms may be within the range described above, but are not limited thereto.

When the composition for producing a retinoid includes the microorganism, it may further include culturing the microorganism in a medium containing a carbon source.

The carbon source may be, for example, selected from the group consisting of starch, glucose, sucrose, galactose, fructose, glycerol, and mixtures thereof, but is not limited thereto.

Hereinafter, the present invention will be described in detail with reference to examples.

### Examples

### Materials and Methods

### 1. Experimental strains and materials

Microorganisms used in the experiment were purchased from ATCC (American Type Culture Collection) and KCCM (Korea Culture Center of Microorganisms) and are summarized in Table 1. The DH5α E. coli strain was used for gene cloning, and the MG1655, AceCo, and SBL7 E. coli strains listed in Table 1 were used to produce the target material. As expression vectors, pTrc99A, pSTV28, and pBBR1MCS2 listed in Table 2 were used. Restriction enzymes and other enzymes used were products from New England Biolabs (USA). Additionally, to perform PCR, Pfu-X DNA polymerase from Solgent (Korea) and Phusion DNA polymerase from Thermo Scientific (USA) were used. The DNA size maker used was a product from Invitrogen (USA). L(+)-arabinose was from Sigma (USA), glycerol was from Amresco (USA), and dodecane was from Honeywell (USA). Other reagents were used from Sigma.

**[Table 1]**

| E. coli strain | explanation |
|---|---|
| DH5α | F⁻f80d*lacZ*DM15D(*lacZYA-argF*)U169 *deoR recA*1 *endA*1 *hsdR*17(r_{K}⁻, m_{K} ⁺) *phoA supE*44I⁻ thi-¹ *gyrA*96 *relA*1 |
| MG1655 | F-λ-*ilvGrfb*-50 *rph*-1 |
| AceCo | *E. coli* MG1655Δ*ackA-pta, poxB, ldhA, dld, adhE, pps, atoDA* |
| SBL7 | *E. coli* MG1655Δ *ackA*-*pta*(::PcTrc-EFAES-ter), *adhE* |
| | (::PcTrc-SN12Didi-ter), *ldhA*(::PcTrc-LYCm4-ter), *atoDA* |
| | (::PcTrc-LYCm4-ter), *dld*(::PcTrc-LYCm4-ter), *poxB* |
| | (::PcTrc-LYCm4-ter), *pps*(::PcTrc-LYCm4-ter) |

**[Table 2]**

| plasmid | Explanation | Information or references (Genbank accession no.) |
|---|---|---|
| pTrc99A | pMB1 origin, trc promoter, laclq, and bla | (U13872.1) |
| pSTV28 | p15A origin, lac promoter, lacZ, and cat | (M22744) |
| pBBR1MCS2 | Broad-host-range, Kmr; lacPOZ mobRP | Kovach et al. 1995 |

The preparation of medium for microbial culture followed the recommended medium composition of each strain distribution organization and the Difco manual (11th edition, Difco; BD Science, USA). Reagents used to prepare media were purchased from BD Science (USA) and Sigma. The amount of bacterial cells during culture was expressed as the result of measuring OD (optical density) at 600 nm using a spectrophotometer (Shimadzu UV-1601, Japan), and pH was measured using a pH meter B-212 (HORIBA, Japan).

For gene cloning, ampicillin, chloramphenicol, and kanamycin were used as antibiotics for plasmid maintenance at concentrations of 100 µg/mL, 50 µg/mL, and 50 µg/mL, respectively.

### 2. Extraction and analysis of retinoids

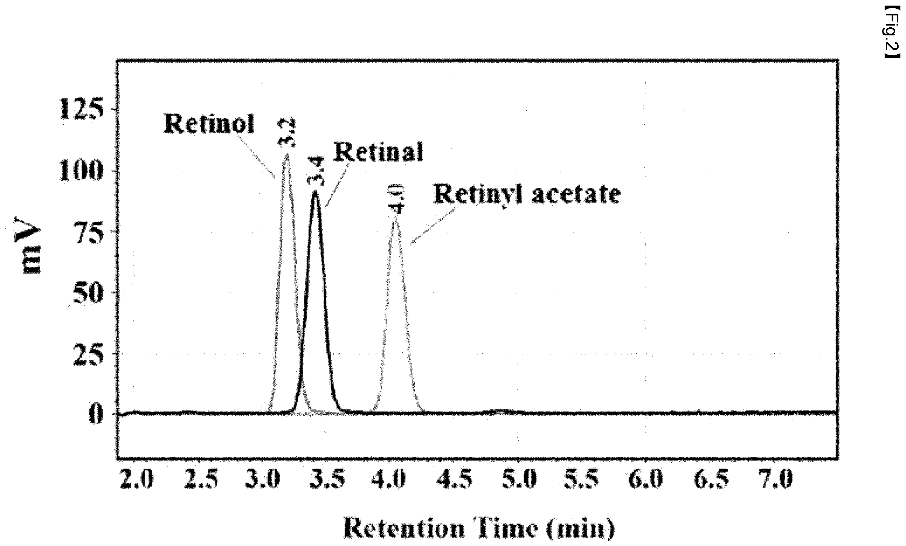

Retinoids were analyzed by the following method. Quantitative HPLC analysis was carried out by taking only the dodecane layer added to the retinoid culture. The retinoid analysis system used is the SHIMADZU LC-20A series with UV/Vis detector (Shimadzu, Kyoto, Japan), and the analysis column was Symmetry C18 (250x4.6,5 µm) with Symmetry guard C18 (15x4.6, 5 µm) was used. The mobile phase was analyzed with a methanol:acetonitrile mixed solution (95:5, v/v) for a total of 15 minutes. The flow rate was set at 1.5 mL/min, the detection wavelength was measured at 370 nm for retinal and 340 nm for retinol and retinyl acetate, the sample injection volume was 20 µℓ, and the oven temperature was 40°C. The retinoid standard sample was dissolved in ethanol and used. As shown in Figure 2, the peak retention time of standard time is about 3.2 minutes for retinol, about 3.4 minutes for retinal, and about 4.0 minutes for retinyl acetate, and the analysis results are based on a calibration curve calculated by calculating the area of the analyzed peak from the peak area of the standard sample. It was calculated by substituting and converting the dilution factor.

### Plasmid, strain development and culture methods

### 1. Construction of plasmid based on cytochrome P450 system

Genes encoding enzymes involved in the cytochrome P450 system are listed in Table 3. Primer sequences and restriction enzymes used for gene cloning are shown in Table 4. The genes were amplified through polymerase chain reaction (PCR) using the primers listed in Table 4 and using the chromosomal DNA of the MG1655 strain containing the corresponding gene as a template. The amplified products were introduced into each vector using the restriction enzymes listed in Table 4, producing vectors pB2-fA, pB2-fB, pB2-fx, pB2-fr, pB2-yK, pB2-fAfr, pB2-fBfr, pB2. -fxfr, pB2-fAyK, pB2-fByK, and pB2-fxyK were constructed.

**[Table 3]**

| Enzyme name | gene | Amino acid sequence (Genbank accession number) |
|---|---|---|
| Flavodoxin 1 | *fldA* | SEQ ID NO: 2 |
| | | NP_415210.1 |
| Flavodoxin 2 | *fldB* | SEQ ID NO: 3 |
| | | NP_417371.1 |
| reduced ferredoxin | *fdx* | SEQ ID NO: 4 |
| | | NP_417020.1 |
| Flavodoxin/ferredoxin-NADP(+) reductase | *fpr* | SEQ ID NO: 5 |
| | | NP_418359.1 |
| Pyruvate-flavodoxin oxidoreductase | *ydbK* | SEQ ID NO: 6 |
| | | NP_415896.1 |

**[Table 4]**

| Vector | Insert | | | restriction enzyme | construction vector |
|---|---|---|---|---|---|
| | gene | primer sequence | | | |
| pBBR1MCS2 | *fldA* | Fwd | SEQ ID NO: 7 | Xbal | pB2-fA |
| | | Rev | SEQ ID NO: 8 | Sacl | |
| pBBR1MCS2 | *fldB* | Fwd | SEQ ID NO: 9 | BamHI | pB2-fB |
| | | Rev | SEQ ID NO: 10 | Xbal | |
| pBBR1MCS2 | *fdx* | Fwd | SEQ ID NO: 11 | Xbal | pB2-fx |
| | | Rev | SEQ ID NO: 12 | Sacl | |
| pBBR1MCS2 | *fpr* | Fwd | SEQ ID NO: 13 | Kpnl | pB2-fr |
| | | Rev | SEQ ID NO: 14 | Sall | |
| pBBR1MCS2 | *ydbK* | Fwd | SEQ ID NO: 15 | Sall | pB2-yK |
| | | Rev | SEQ ID NO: 16 | Xbal | |
| pB2-fA | *fpr* | Fwd | SEQ ID NO: 13 | Kpnl | pB2-fAfr |
| | | Rev | SEQ ID NO: 14 | Sall | |
| pB2-fB | *fpr* | Fwd | SEQ ID NO: 13 | Kpnl | pB2-fBfr |
| | | Rev | SEQ ID NO: 14 | Sall | |
| pB2-fx | *fpr* | Fwd | SEQ ID NO: 13 | Kpnl | pB2-fxfr |
| | | Rev | SEQ ID NO: 14 | Sall | |
| pB2-fA | *ydbK* | Fwd | SEQ ID NO: 15 | Sall | pB2-fAyK |
| | | Rev | SEQ ID NO: 16 | Xball | |
| pB2-fB | *ydbK* | Fwd | SEQ ID NO: 15 | Sall | pB2-fByK |
| | | Rev | SEQ ID NO: 17 | HindIII | |
| pB2-fx | *ydbK* | Fwd | SEQ ID NO: 15 | Sall | pB2-fxyK |
| | | Rev | SEQ ID NO: 16 | Xball | |

### 2. Development of strains with improved lycopene production efficiency

This example includes contents related to the production of an E. coli MG1655 strain with improved productivity of lycopene, a beta-carotene precursor, to improve retinoid production.

In this example, in order to improve the efficiency of lycopene production, genes involved in the production of organic acids and alcohols, which are fermentation by-products, were deleted in strain MG1655, and the exogenous mevalonate (MVA) pathway and lycopene biosynthetic pathway were introduced multiple times into the E. coli chromosome. In order to increase the expression of isoprenoid biosynthesis genes on the E. coli chromosome, the original promoters of rate-limiting genes were replaced with strong trc promoters modified to ensure constant expression, and the final SBL7 strain was created.

### 2-1. Production of strains introducing foreign MVA upstream/downstream pathways into the E. coli chromosome

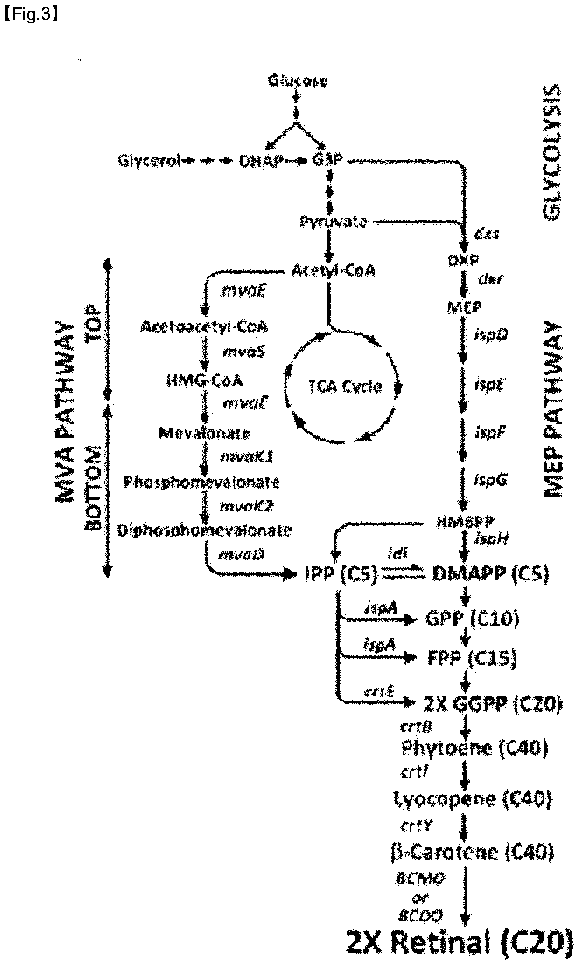

Describing the production process of the SBL7 strain in detail, first, the MVA pathway in Figure 3 was divided into an upper pathway from acetyl-CoA to mevalonate based on mevalonate and a lower pathway synthesizing mevalonate through IPP to DMAPP. These pathways were inserted into the chromosome of E. coli MG1655 strain using the high-efficiency genetic resources of the exogenous MVA pathway.

The genes that form the upper mevalonate pathway are *mvaE* (SEQ ID NO: 18) and *mvaS* (SEQ ID NO: 19) derived from *Enterococcus faecalis* PCI1326, and the pTFKC (DPB) vector (Korean Patent Publication No. 2018-0124777) is used to insert the two genes into E. coli. pTFKC(DPB)-EFAES was constructed by cloning using the PCR primers in Table 5 (Table 6).

The insertion site upstream of the MVA pathway was inserted by simultaneously deleting the *ackA* gene, an acetate kinase involved in acetate production, and the *pta* gene, a phosphate acetyltransferase/phosphate propionyltransferase. For gene deletion, the homologous recombination method using Arecombinase was used. The prepared MVA upper pathway insertion strain was named APTOP and is shown in Table 7. At this time, the promoter included the trc promoter and terminator modified to ensure constant expression, so the inserted upper path was designated as PcTrc-EFAES-ter.

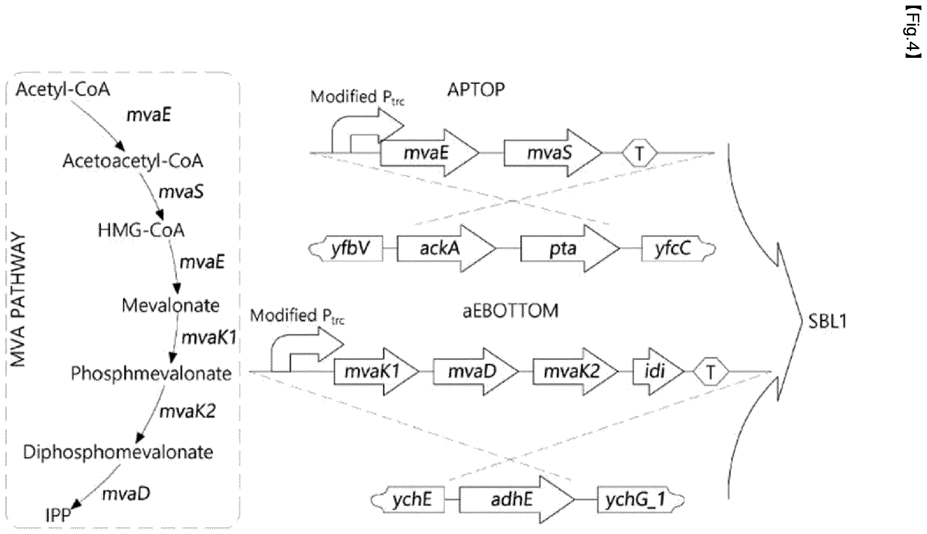

The MVA lower pathway consists of mvaK1 (SEQ ID NO: 20), mvaK2 (SEQ ID NO: 21), mvaD (SEQ ID NO: 22) of Streptococcus pneumoniae and idi (SEQ ID NO: 23) genes of Escherichia coli, and the genes of the above genes were amplified using the primer in Table 5 so that four genes could be inserted into Escherichia coli, and pTFCC (DPB) - SN12Didi was constructed by cloning it on a pTFCC (DPB) vector (Korean Open Patent No. 2018-0124777) (Table 6). The MVA lower pathway was inserted at the same time as the adhE gene defect, and was inserted using a primer in Table 5 having a homology of 50 bp to the E. coli chromosome to be inserted according to the gene. For gene defects, homologous recombination method using Arecombinase was used. Through this, MVA subpathway insertion strain was constructed. In addition, since the promoter at the time of insertion includes a modified trc promoter and a terminator to always be expressed, the inserted upper path is labeled as PcTrc-SN12Didi-ter. This was selected as a mevalonate subpathway insertion strain and named aEBOTTOM (Table 7). An E. coli strain containing the entire MVA pathway was developed using the P1 transduction method using the strain inserting the MVA upper/lower pathway constructed above, and was named SBL1 (FIG. 4, Table 7).

**[Table 5]**

| MVA path | primer | sequence number | MVA path | primer | sequence number |
|---|---|---|---|---|---|
| upper pathway | EFAES-F | 24 | lower pathway | IS adhE-Ptrc-F | 36 |
| | EFAES-R | 25 | | KO adhE-R | 37 |
| | IS ackA-pta-Ptrc-F | 26 | | IS atoDA-Ptrc-F | 38 |
| | KO ackA-pta-R | 27 | | Also DA-R | 39 |
| | KO | 28 | | KO poxBCF-F | 40 |
| | ackA-ptaCF-F | | | | |
| | KO ackA-ptaCF-R | 29 | | KO poxBCF-R | 41 |
| lower pathway | SN12 Didi-F | 30 | | OR IdhACF-F | 42 |
| | SN12 Didi-R | 31 | | OR IdhACF-R | 43 |
| | IS poxB-Ptrc-F | 32 | | KO adhECF-F | 44 |
| | THE poxB-R | 33 | | KO adhECF-R | 45 |
| | IS IdhA-Ptrc-F | 34 | | OR atoDACF-F | 46 |
| | KO IdhA-R | 35 | | OR atoDACF-R | 47 |

**[Table 6]**

| plasmid | explanation |
|---|---|
| | pTFKC(DPB) |
| pTFKC(DPB)-EFAES | containing *mvaE* and *mvaS* of *Enterococcus* |
| | *faecalis* PCI1326 |
| | pTFCC(DPB) |
| pTFCC(DPB)-SN12Didi | containing *mvaK1*, *mvak2* and *mvaD* of *S.* |
| | *pneumonia*, and *idi* of *E. coli* |

**[Table 7]**

| strain | explanation |
|---|---|
| APTOP | MG1655Δ *ackA* -pta(::PcTrc-EFAES-ter) |
| aEBOTTOM | MG1655Δ*adhE* (::PcTrc-SN12Didi-ter) |
| SBL1 | MG1655Δ *ackA* -pta(::PcTrc-EFAES-ter), adhE(::PcTrc-SN12Didi-ter) |

### 2-2. Construction of a strain with multiple insertion of lycopene biosynthetic operon within the E. coli chromosome

A total of four genes, *crtE* (SEQ ID NO: 48), *crtB* (SEQ ID NO: 49), and *crtl* (SEQ ID NO: 50) genes of *Pantoea agglomerans*, which are lycopene biosynthesis pathways, and *ipiHP1* (SEQ ID NO: 52) genes of Haematococcus pluvialis, were cloned into the pBFKC vector (Korean Patent No. 2018-0124777) to construct pBFKC-LYCm4 (Table 8) to be able to insert the four genes into E.coli. Using the constructed plasmid as a template, the primer of Table 9 having a homology of 50 bp was inserted into the E. coli chromosome to be inserted according to each gene. The genes for inserting lycopene biosynthetic operons into the Escherichia coli chromosomes were selected for *poxB*, *ldhA*, and *dld* genes to block the production of acetate and lactate, pps genes for pyruvate preservation, and *atoD* and *atoA* genes for acetyl-CoA preservation. Therefore, in the MG1655 strain, the above genes were defected by homologous recombination method using Arecombinase, and lycopene biosynthetic operon containing trc promoter was inserted. As a result, a total of 5 strains with lycopene biosynthetic operon were constructed, which were named MG1655ΔldhA (::P trc-LYCm4-ter), MG1655ΔatoDA (::P trc-LYCm4-ter), MG1655Δdld (::P trc-LYCm4-ter), MG1655ΔpoxB (::P trc-LYCm4-ter), and MG1655Δpps (::P trc-LYCm4-ter).

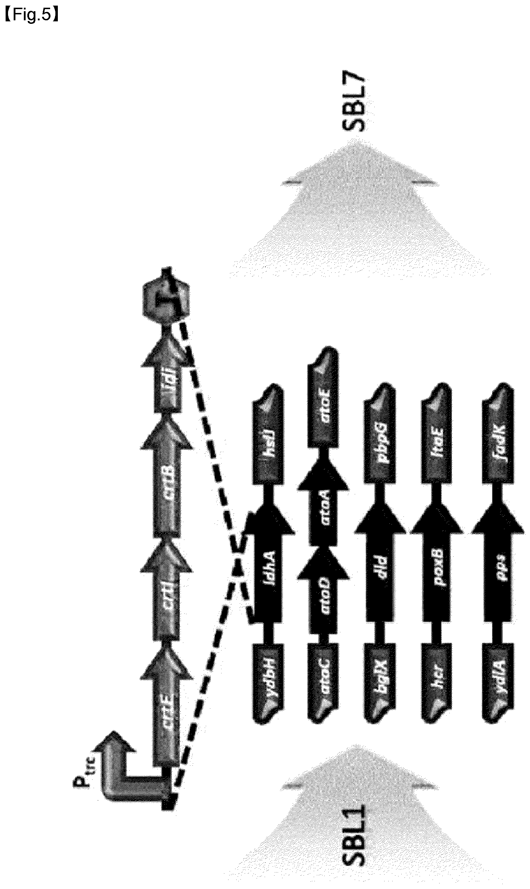

Next, lycopene biosynthetic operons were inserted into the SBL1 strain with the foreign MVA full pathway inserted into the Escherichia coli chromosome, and lycopene operons were sequentially multiplexed into the SBL1 strain through P1 transduction from strains inserted at *ldhA*, *atoDA, dld*, *poxB*, and *pps* gene sites, which was named SBL7 (FIG. 5, Table 10).

**[Table 8]**

| plasmid | explanation |
|---|---|
| pBFKC-LYCm4 | pBFKC containing *crtE, crtB* and *crtl of P. agglomerans*, and *ipiHP1* of *H. pluvialis* |

**[Table 9]**

| primer | sequence number | primer | sequence number |
|---|---|---|---|
| LYCm4-F | 53 | KO pps-R | 64 |
| LYCm4-R | 54 | KO poxBCF-F | 65 |
| IS poxB-Ptrc-F | 55 | KO poxBCF-R | 66 |
| KO poxB-R | 56 | KO IdhACF-F | 67 |
| IS IdhA-Ptrc-F | 57 | KO ldhACF-R | 68 |
| KO IdhA-R | 58 | KO dldCF-F | 69 |
| IS dld-Ptrc-F | 59 | KO dldCF-R | 70 |
| KO dld-R | 60 | KO atoDACF-F | 71 |
| IS atoDA-Ptrc-F | 61 | KO atoDACF-R | 72 |
| KO atoDA-R | 62 | KO ppsCF-F | 73 |
| IS pps-Ptrc-F | 63 | KO ppsCF-R | 74 |

**[Table 10]**

| strain | explanation |
|---|---|
| SBL7 | SBL1ΔldhA(::Ptrc-LYCm4-ter), atoDA(::Ptrc-LYCm4-ter), dld(::Ptrc-LYCm4-ter), poxB(::Ptrc-LYCm4-ter), pps(::Ptrc-LYCm4-ter) |

From the above results, E. coli with lycopene productivity was developed through multiple insertions of the lycopene biosynthetic operon into the chromosome. Since this E. coli strain itself has the ability to produce lycopene, it can stably produce lycopene without the introduction of a plasmid.

### 2-3. Production of plasmid introduction strain based on cytochrome P450 system

E. coli MG1655, AceCo (Korean Open Patent No. 2017-0089426), including the retinoid-producing plasmid pT-DHBSR and the MVA pathway expressing plasmid pS-NA (PCT Patent No. 2013-019051), and the SBL7 strain produced in 2-2 above are transformed to produce an E. coli transformant.

Competent cells are fabricated to transform plasmids into MG1655 (pT-DHBSR / pS-NA), AceCo (pT-DHBSR / pS-NA), and SBL7 (pT-DHBSR / pS-NA) strains. MG1655 (pT-DHBSR / pS-NA), AceCo (pT-DHBSR / pS-NA), and SBL7 (pT-DHBSR / pS-NA) strains are inoculated in 5 ml LB medium containing ampicillin and chloramphenicol antibiotics, and OD600, 2-3 at temperature 30 ° C. 5 ml of new LB medium containing Ampicillin and chloramphenicol antibiotics will be incubated at 30 ° C. for 3 hours after inoculation to OD600, 0.1. To make water-soluble cells, the culture medium is cooled in ice for 5 minutes, centrifuged for 5 minutes at a temperature of 4 ° C. and 5000 rpm for 5 minutes to remove the supernatant, and washed twice with 1 ml of washing buffer (0.5 M sucrose, 10% glycerol). The washed cells are suspended in 0.1 ml of washing buffer to obtain water-soluble cells.

Add 100 ng/µl of the recombinant plasmid to 100 µl of the obtained soluble cells, mix, place the mixture in an electroporation cuvette (2 mm apart), and perform electroporation under the conditions of 25 µF, 200 Ω, and 2,500 V. After electroporation, add 1 ml of LB medium and incubate at 37°C for 1 hour. Transformants are obtained by plating 100 µl of the cell sample into which the recombinant plasmid was introduced by electroporation on LB solid plate medium containing ampicillin, chloramphenicol, and kanamycin antibiotics.

### 2-4. Retinoid culture methods and results

The above-constructed transformant was inoculated into 5 ml of 2YT medium containing ampicillin, chloramphenicol, and kanamycin antibiotics, and seed culture was performed at a temperature of 37°C to OD₆₀₀, 2-3 After adding 2% (w/v) glycerol and 0.2% (w/v) L(+)-arabinose as a carbon source to 5 ml of new 2YT medium containing ampicillin, chloramphenicol, and kanamycin antibiotics, adjust to OD 600, 0.1 . After inoculation, 5 ml of dodecane was overlaid and cultured in a shaking incubator at 30°C and 250 rpm for 72 hours to confirm retinoid productivity.

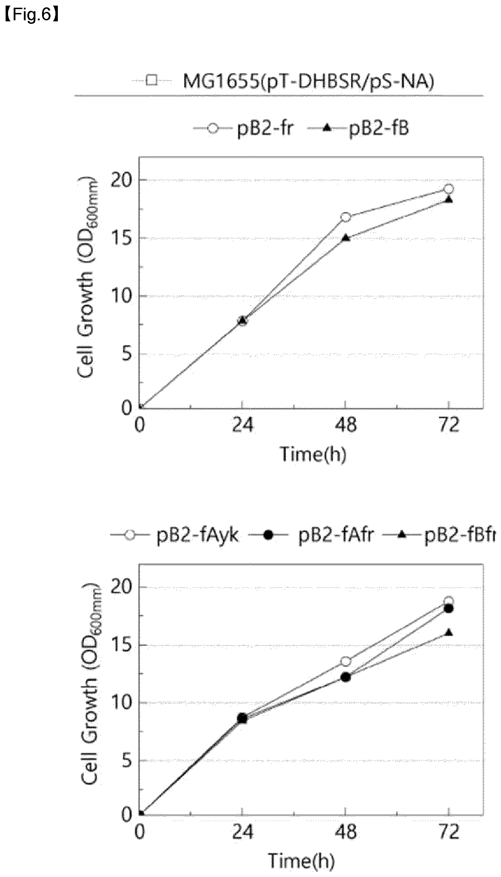

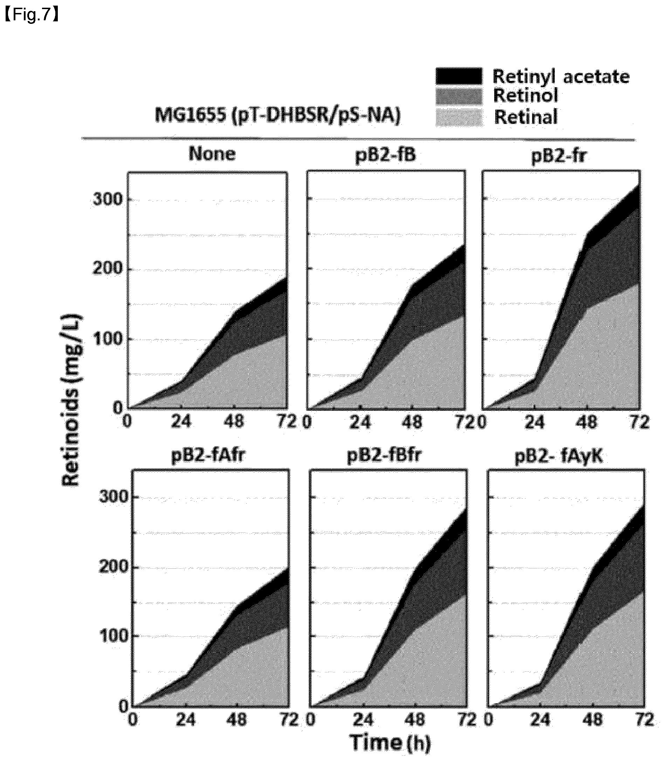

The results of retinoid culture in which a plasmid based on the cytochrome P450 system was introduced into the MG1655 strain and activated the cytochrome P450 system are shown in Table 11 and FIG. 6 and 7. In detail, bacterial growth was similar in all strains, and retinoid production was 322 mg/L when the *fpr* gene was overexpressed, which was about 1.7-fold higher than the MG1655 strain containing pT-DHBSR and pS-NA produced 190 mg/L. As a result of overexpression in various combinations, retinoid production increased to 288 mg/L and 294 mg/L when fldB-fpr and fldA-ydbK genes were overexpressed.

**[Table 11]**

| Strain | MG1655 (pT-DHBSR/pS-NA) | | | | | |
|---|---|---|---|---|---|---|
| (72hr) | None | pB2-fB | pB2-fr | pB2-fAfr | pB2-fBfr | pB2-fAyK |
| Cell growth amount (OD₆₀₀) | 16.7±0.4 | 18.3±0.3 | 19.2±0.2 | 16.8±0.3 | 16.0±0.2 | 18.4±1.8 |
| Total retinoids (mg/L) | 190 | 236 | 322 | 201 | 288 | 294 |

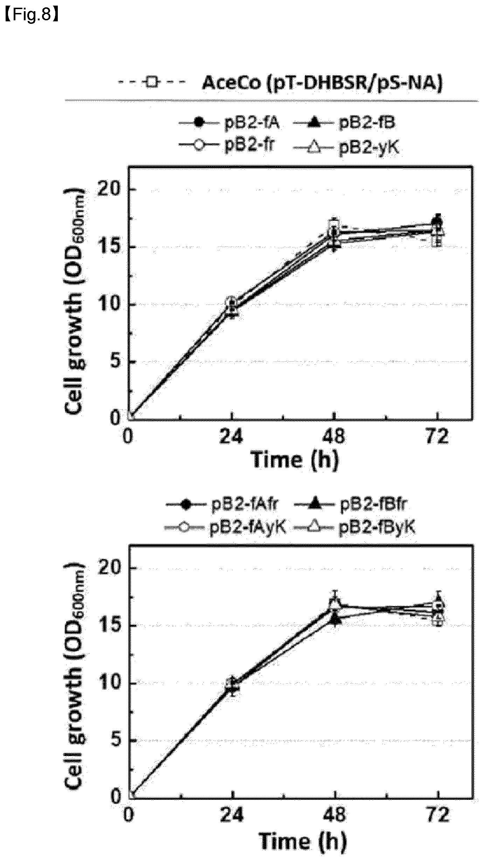

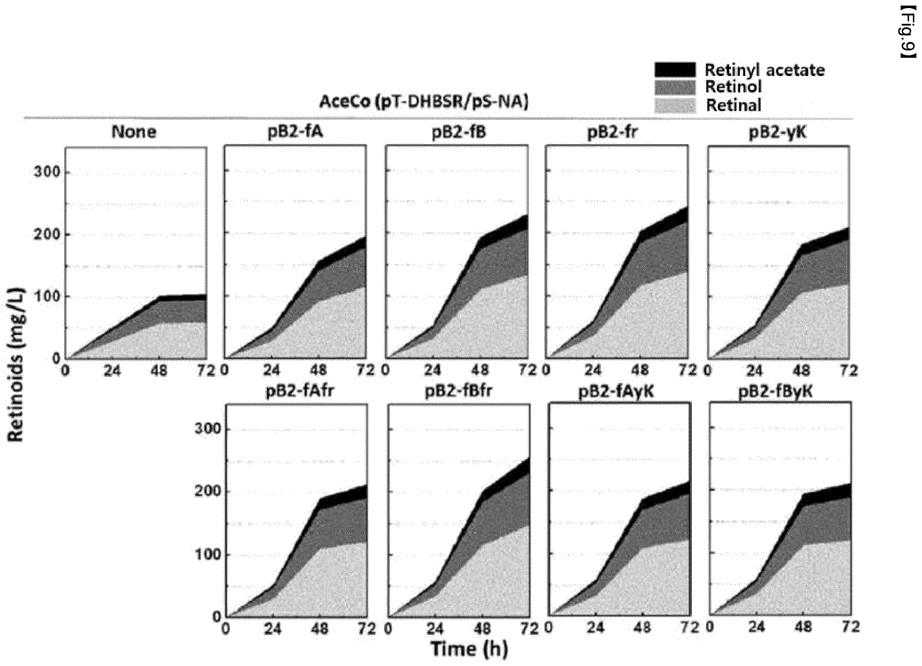

The results of retinoid culture in which a plasmid based on the cytochrome P450 system was introduced into the AceCo strain and activated the cytochrome P450 system are shown in Table 12 and FIGS. 8 and 9. In detail, cell growth was similar in all strains, and when the *fldA*, *fldB*, *fpr*, and *ydbK* genes were overexpressed, the retinoid production increased to 196 mg/L, 230 mg/L, 243 mg/L, and 211 mg/L, respectively, compared to 101 mg/L produced the AceCo strain containing conventional pT-DHBSR and pS-NA at 72 hours of incubation. Overexpression of *fldA-fpr*, *fldB-fpr*, *fldA-ydbK*, and *fldB-ydbK* genes resulted in an increase in retinoid production to 212 mg/L, 256 mg/L, 216 mg/L, and 212 mg/L, respectively.

**[Table 12]**

| Strain (72hr) | AceCo (pT-DHBSR/pS-NA) | | | | | | | | |
|---|---|---|---|---|---|---|---|---|---|
| | None | pB2-fA | pB2-fB | pB2-fr | pB2-yK | pB2-fAfr | pB2-fBfr | pB2-fAy K | pB2-fBy K |
| Cell growth amount (OD₆₀₀) | 15.5± 0.1 | 17.1± 0.6 | 16.4± 1.0 | 16.5± 1.3 | 16.5± 0.1 | 16.2± 0.6 | 17.1± 2.3 | 16.8± 0.5 | 15.8± 0.8 |
| Total retinoids (mg/L) | 101 | 196 | 230 | 243 | 211 | 212 | 256 | 216 | 212 |

The results of retinoid culture in which a plasmid based on the cytochrome P450 system was introduced into the SBL7 strain and activated the cytochrome P450 system are shown in Tables 13 and 10 and 11. In detail, cell growth was similar in all strains, and when the *fldA*, *fldB*, and *fdx* genes were overexpressed, retinoid production increased to 471 mg/L, 467 mg/L, and 422 mg/L, respectively, compared to 310 mg/L produced by the SBL7 strain containing pT-DHBSR and pS-NA at 72 hours. As a result of overexpression in various combinations, retinoid production increased to 481 mg/L and 475 mg/L when *fldB-fpr* and *fldB-ydbK* genes were overexpressed. In particular, the highest retinoid productivity was shown at 481 mg/L when the *fldB-fpr* gene was overexpressed, and the production was increased by about 1.6 times compared to the SBL7 strain containing the existing pT-DHBSR and pS-NA.

**[Table 13]**

| Strain (72hr) | SBL7 (pT-DHBSR/pS-NA) | | | | | | | |
|---|---|---|---|---|---|---|---|---|
| | None | pB2-fA | pB2-fB | pB2-fx | pB2-fBfr | pB2-fxfr | pB2-fByK | pB2-fxyK |
| Cell growth | 18.1± 0.1 | 18.1± 0.1 | 19.0± 0.0 | 17.8± 0.1 | 17.9± 0.6 | 19.0± 0.4 | 18.1± 0.5 | 17.7± 0.1 |
| amount (OD₆₀₀) | | | | | | | | |
| Total retinoids (mg/L) | 310 | 471 | 467 | 422 | 481 | 380 | 475 | 339 |

Based on the above results, the effect on retinoid production was different when *fldA*, *fldB*, *fdx*, *fpr*, and *ydbK* genes were overexpressed in each strain of MG1655, AceCo, and SBL7, either in combination or in combination, but the efficiency increase of the cytochrome P450 system improved retinoid productivity.

## Claims

1. A composition for production of retinoid comprising a NADH or NADPH, a Beta-Carotene Oxygenase and a flavodoxin or a ferredoxin or a reductase of flavodoxin or ferredoxin; or a microorganism expressing them; or a culture or pulverized product of the microorganism.

2. The composition for production of retinoid according to claim 1, wherein the Beta-Carotene Oxygenase is a Beta-Carotene MonoOxygenase(BCMO, Beta-Carotene 15,15'-MonoOxygenase) comprising a sequence of SEQ ID NO: 1.

3. The composition for production of retinoid according to claim 1, wherein the flavodoxin is a flavodoxin 1(fldA) comprising a sequence of SEQ ID NO: 2 or a flavodoxin 2(fldB) comprising a sequence of SEQ ID NO: 3.

4. The composition for production of retinoid according to claim 1, wherein the ferredoxin is a reduced ferredoxin(fdx) comprising a sequence of SEQ ID NO: 4.

5. The composition for production of retinoid according to claim 1, wherein the flavodoxin or ferredoxin reductase is a flavodoxin/ferredoxin-NADP(+) reductase(fpr) comprising a sequence of SEQ ID NO: 5 or a pyruvate-flavodoxin oxidoreductase(YdbK) comprising a sequence of SEQ ID NO: 6.

6. The composition for production of retinoid according to claim 1, wherein the microorganism is of the genus *Escherichia.*

7. The composition for production of retinoid according to claim 1, wherein the microorganism further expresses a gene encoding an enzyme of a mevalonate(MVA) synthesis pathway or a beta-carotene synthesis pathway.

8. The composition for production of retinoid according to claim 7, wherein the enzyme of the mevalonate synthesis pathway is an acetyl-CoA acetyltransferase/hydroxymethylglutaryl (HMG)-COA reductase(mvaE) comprising a sequence of SEQ ID NO: 18, a HMG-CoA synthase(mvaS) comprising a sequence of SEQ ID NO: 19, a mevalonate kinase(mvaK1) comprising a sequence of SEQ ID NO: 20, a phosphomevalonate kinase(mvaK2) comprising a sequence of SEQ ID NO: 21, a mevalonate diphosphate decarboxylase(mvaD) comprising a sequence of SEQ ID NO: 22, and an isopentenyldiphosphate isomerase(idi) comprising a sequence of SEQ ID NO: 23.

9. The composition for production of retinoid according to claim 7, wherein the enzyme of the beta-carotene synthesis pathway is a geranylgeranyl pyrophosphate synthase(crtE) comprising a sequence of SEQ ID NO: 48, a phytoene synthase(crtB) comprising a sequence of SEQ ID NO: 49, a phytoene dehydrogenase(crtl) comprising a sequence of SEQ ID NO: 50, a lycopene beta-cyclase(crtY) comprising a sequence of SEQ ID NO: 51, an IPP isopomerase(isopentenyl pyrophosphate:dimethylallyl pyrophosphate isomerase, ipiHP1) comprising a sequence of SEQ ID NO: 52.

10. The composition for production of retinoid according to claim 1, wherein the microorganism where at least one gene encoding an enzyme selected from a group consisting of ackA(acetate kinase), pta(phosphate acetyltransferase/phosphate propionyltransferase), adhE(aldehyde-alcohol dehydrogenase), IdhA(lactate dehydrogenase A), atoDA(acyl CoA:acetate/3-ketoacid CoA transferase), dld(D-lactate dehydrogenase), poxB(pyruvate dehydrogenase) and pps(phosphoenolpyruvate synthase) is attenuated or deleted.

11. A method for production of retinoid comprising reacting a beta-carotene with the composition according to claim 1.

12. The method for production of retinoid according to claim 11, wherein the composition comprises the microorganism, and the reaction is carried out within the microorganism.

13. The method for production of retinoid according to claim 11, wherein the composition comprises the microorganism, and the method further comprises culturing the microorganism in a medium containing a carbon source.
